# EUROPÄISCHE PATENTSCHRIFT

(11) **EP 0 840 079 B1**
(45) Veröffentlichungstag und Bekanntmachung des Hinweises auf die Patenterteilung: **13.02.2002**
(21) Anmeldenummer: 97117620.1
(22) Anmeldetag: 10.10.1997
(51) Int. Cl.: F25J 3/06, C07C 7/00

(54) **Verfahren zur Tieftemperaturzerlegung eines im Wesentlichen aus Wasserstoff, Methan und C3-, C4- oder C3/C4- Kohlenwasserstoffen bestehenden Stromes**
Cryogenic separation process of streams containing hydrogen methane, C3, C4, or C3/C4 hydrocarbons
Procédé de séparation cryogénique de courants contenant de l'hydrogène, méthane, C3-, C4- ou C3/C4 hydrocarbures

(30) Priorität: 31.10.1996 DE 19644106
(43) Veröffentlichungstag der Anmeldung: 06.05.1998
(73) Patentinhaber: Linde Aktiengesellschaft, 65189 Wiesbaden (DE)
(72) Erfinder: Bauer, Heinz, Dr. Dipl.-Phys., 82067 Ebenhausen (DE)
(74) Vertreter: Zahn, Christoph

(56) Entgegenhaltungen:
- DE-A- 4 235 006

## Beschreibung

Die Erfindung betrifft ein Verfahren gemäß dem Oberbegriff des Patentanspruches 1.

Verfahren zur Tieftemperaturzerlegung eines im wesentlichen aus Wasserstoff, Methan und C₃-, C₄- oder C₃/C₄-Kohlenwasserstoffen bestehenden Stromes finden z.B. bei der der Dehydrierung von Iso-Butan zu Iso-Buten nachgeschalteten kryogenen Zerlegung des aus dem Dehydrierreaktor stammenden Produktstromes Verwendung. Die Dehydrierung von Iso-Butan zu Iso-Buten spielt vor allem bei der Herstellung von MTBE (Methyl-tert.-Butylether) eine wichtige Rolle, da zur Herstellung von MTBE Iso-Buten und Methanol benötigt werden. Aufgrund von sich zunehmend verschärfenden Abgasvorschriften für Kraftfahrzeuge wird der Verbrauch an MTBE, das als Oktanzahl-Verbesserer für Kraftstoffe verwendet wird, in den folgenden Jahren ansteigen. Während Methanol in ausreichenden Mengen zur Verfügung steht, besteht jedoch an Iso-Buten ein erheblicher Mangel.

Einen Überblick über die zum Stand der Technik zählenden Verfahrensmöglichkeiten zur Gewinnung von C₃/C₄-Kohlenwasserstoff-reichen Produktfraktionen gibt der Artikel "Cryogenic Olefins Recovery from Dehydrogenation Reactor Effluents" von Dr. Heinz C. Bauer (präsentiert auf dem AlChE-Symposium on Cryogenic Gas Processing, 1992 Spring National Meeting, 29.03. - 02.04.92).

Insbesondere Figur 13 zeigt hierbei ein Verfahren zum Zerlegen eines im wesentlichen aus Wasserstoff, Methan und C₄-Kohlenwasserstoffen bestehenden Stromes, bei dem ein Teil des gewonnenen Gasgemisches, bestehend aus Wasserstoff und Methan, aus der Anlage abgeführt (Net fuel gas) und ein Teil in den Dehydrierreaktor (Recyclegas) zurückgeführt wird. Diese Rückführung eines Teiles des Gasgemisches in den Dehydrierreaktor geschieht, um in ihm eine unerwünschte Koksbildung zu vermindern. Dies gelingt jedoch nur unvollständig. Es wurde zwischenzeitlich festgestellt, daß sich die unerwünschte Koksbildung umso besser unterdrücken läßt, je höher der Wasserstoffanteil und je niedriger der Gehalt an Olefinen in dem zu dem Dehydrierreaktor zurückgeführten Strom ist. Zusätzliche Stoffe, wie z. B. Methan, stellen nur einen unnötigen Ballast dar, der sowohl die Kapazität blockiert als auch die Wirtschaftlichkeit des Verfahrens verschlechtert.

Die der bzw. aus der Tieftemperaturzerlegung zu- bzw. abgeführten Verfahrensströme werden oftmals in lediglich einem Wärmetauscher abgekühlt und teilkondensiert bzw. angewärmt und verdampft. Bekannt ist ferner die Aufteilung der Verfahrensströme auf zwei Wärmetauscher, wobei jedoch der Wärmetauscher, in dem die Abkühlung und Teilkondensation des im wesentlichen aus Wasserstoff, Methan und C₃-, C₄- oder C₃/C₄-Kohlenwasserstoffen bestehenden Stromes und die Anwärmung und Verdampfung des C₃-, C₄- oder C₃/C₄-Kohlenwasserstoff-reichen Dehydrierung-Einsatzstromes erfolgt, von wenigstens zwei weiteren Verfahrensströmen, die am Wärmeaustausch zwischen den beiden genannten Verfahrensströmen beteiligt sind, durchströmt wird.

Erfolgt jedoch in der Praxis die Verteilung auf die einzelnen Kanäle des oder gegebenenfalls der Wärmetauscher und die Verdampfung des C₃-, C₄- oder C₃/C₄-Kohlenwasserstoff-reichen Dehydrierungs-Einsatzstromes, dem zumindest ein Teil des Wasserstoff beigemischt ist, nicht nahezu ideal, werden sowohl die Anlagenkapazität als auch die Produktreinheit des aus der Dehydrierung abgezogenen Gasstromes durch einen ungleichmäßigen Wärmeübergang stark beeinträchtigt.

Die Berechnung eines mehrstromigen Wärmetauschers bzw. dessen Realisierung ist jedoch erfahrungsgemäß sehr aufwendig. Insbesondere der Einfluß der sog. Verteilzonen des Wärmetauschers spielt bei zweiphasig zugespeisten Verfahrensströmen eine erheblich Rolle.

Ziel und Aufgabe der vorliegenden Erfindung ist es, ein Verfahren zur Tieftemperaturzerlegung eines im wesentlichen aus Wasserstoff, Methan und C₃-, C₄- oder C₃/C₄-Kohlenwasserstoffen bestehenden Einsatzstromes anzugeben, das die genannten Nachteile vermeidet.

Dies wird erfindungsgemäß durch ein Verfahren mit den Merkmalen des Patentanspruches 1 erreicht.

Eine weitere Ausgestaltung des erfindungsgemäßen Verfahrens ist dadurch gekennzeichnet, daß als Wärmetauscher für den Wärmeaustausch zwischen dem im wesentlichen aus Wasserstoff, Methan und C₃-, C₄- oder C₃/C₄-Kohlenwasserstoffen bestehenden Strom und dem C₃-, C₄- oder C₃/C₄-Kohlenwasserstoff-reichen Dehydrierungs-Strom ein Plattenwärmetauscher, vorzugsweise ein Aluminium-Plattenwärmetauscher, verwendet wird.

Die Erfindung ausgestaltend wird weiterhin vorgeschlagen, daß der zum Zwecke der Kältebereitstellung durch die Tieftemperaturzerlegung geführte, C₃-, C₄- oder C₃/C₄-Kohlenwasserstoff-reiche Dehydrierungs-Strom auf unterschiedlichen Temperatumiveaus mit dem in der Tieftemperaturzerlegung gewonnenen Wasserstoff vermischt wird.

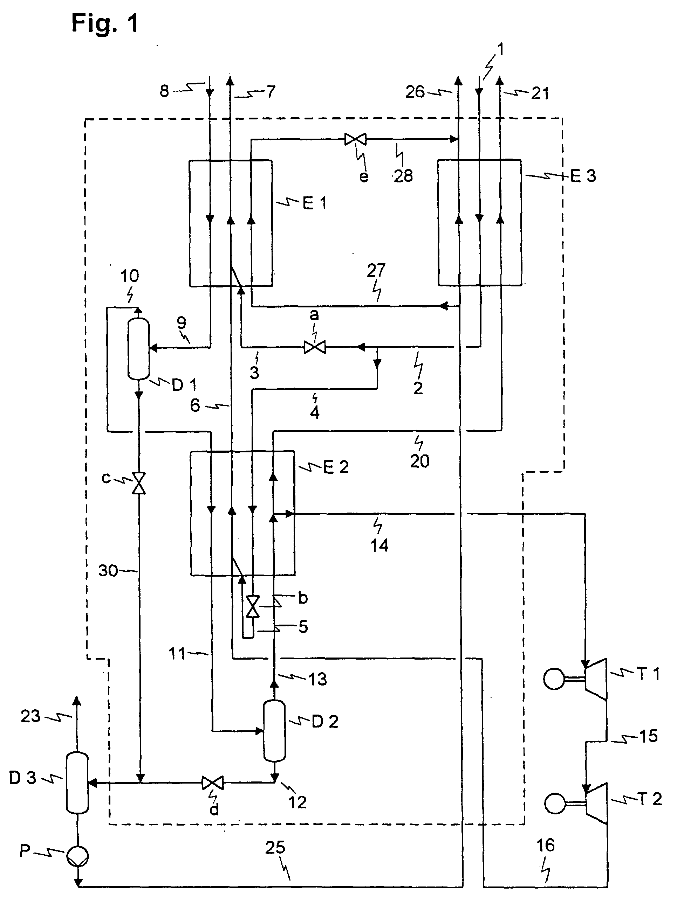

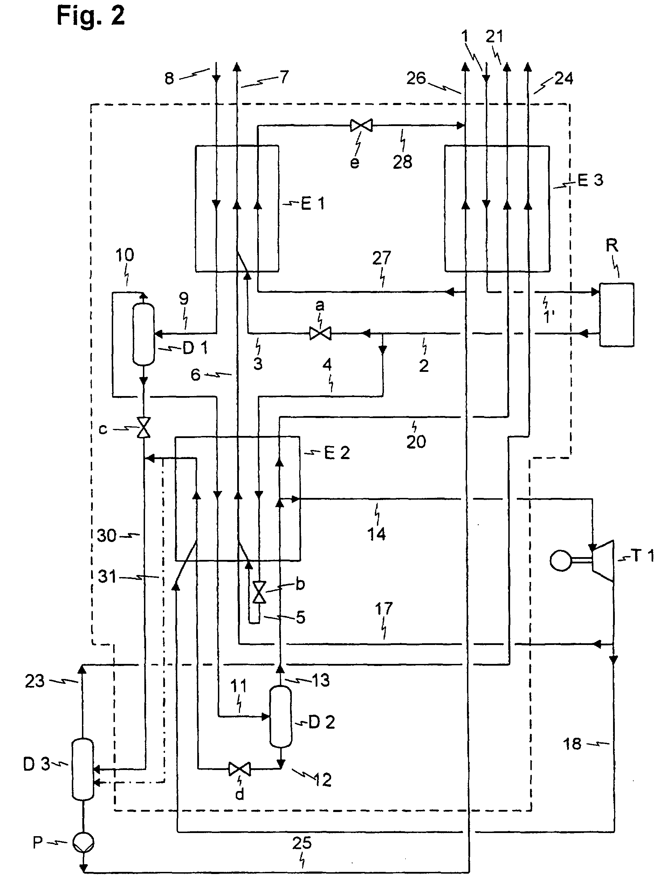

Das erfindungsgemäße Verfahren sowie weitere Ausgestaltungen desselben seien anhand der in den Figuren 1 und 2 dargestellten Ausführungsbeispiele erläutert.

Die in den Figuren 1 und 2 gestrichelt gezeichnete Linie umschließt denjenigen Bereich, der in der Praxis als sog. Cold-Box ausgeführt wird. Nicht dargestellt in den Figuren 1 und 2 sind der bzw. die Dehydrierstufen sowie Verdichtungs- und gegebenenfalls Vorbehandlungsschritte.

**Figur 1** zeigt hierbei ein Verfahren zur Tieftemperaturzerlegung eines im wesentlichen aus Wasserstoff, Methan und C₃-Kohlenwasserstoffen bestehenden Einsatzstromes.

Der der Dehydrierung zugeführte flüssige Kohlenwasserstoff-reiche Einsatzstrom wird über Leitung 1 in die Tieftemperaturzerlegung bzw. den Wärmetauscher E 3 geführt. In diesem wird er gegen anzuwärmende Verfahrensströme unterkühlt. Über Leitung 2 wird dieser Strom aus dem Wärmetauscher E 3 abgezogen und anschließend in zwei Teilströme 3 und 4 aufgeteilt. Beide Teilströme 3 und 4 werden im folgenden mit einem Wasserstoff-reichen Strom vermischt werden.

Dazu wird der erste Teilstrom 3 zunächst im Ventil a entspannt und anschließend vor bzw. im Wärmetauscher E 1 mit dem Wasserstoff-reichen Strom in Leitung 6 vermischt. Der zweite Teilstrom 4 wird im Wärmetauscher E 2 weiter unterkühlt, im Ventil b entspannt und anschließend über Leitung 5 dem Wasserstoff-reichen Gasstrom in Leitung 16 beigemischt. Auf diesen Wasserstoff-reichen Gasstrom in Leitung 6 bzw. 16 sowie dessen Herkunft wird im folgenden noch eingegangen werden.

Der über Leitung 7 aus dem Wärmetauscher E 1 abgezogene Gemischstrom aus dem Wasserstoff- und Kohlenwasserstoff-reichen Strom wird nun einer ein- oder mehrstufigen Dehydrierung zugeführt. Das aus der Dehydrierung abgezogene Reaktionsprodukt wird anschließend ein- oder mehrstufig komprimiert, gegebenenfalls einer Vorbehandlung, wie z. B. Trocknung, HCI-Entfernung, etc., unterworfen und als im wesentlichen aus Wasserstoff, Methan und C₃-Kohlenwasserstoffen bestehender Strom über Leitung 8 wieder der Tieftemperaturzerlegung bzw. dem Wärmetauscher E 1 zugeführt. Dieser Strom wird im Wärmetauscher E 1 im Wärmeaustausch mit anzuwärmenden Verfahrensströmen abgekühlt, teilkondensiert und anschließend über Leitung 9 einem ersten Abscheider D 1 zugeführt.

Am Kopf des Abscheiders D 1 wird über Leitung 10 eine Wasserstoff-reiche Gasfraktion abgezogen, im Wärmetauscher E 2 abgekühlt und teilkondensiert und anschließend über Leitung 11 einem zweiten Abscheider D 2 zugeführt. Aus dem Sumpf des Abscheiders D 1 wird über Ventil c und Leitung 30 eine C₃-Kohlenwasserstoff-reiche Flüssigfraktion abgezogen und in einen dritten Abscheider D 3 geführt.

Aus dem Sumpf des Abscheiders D 2 wird über Leitung 12 und Ventil d ebenfalls eine C₃-Kohlenwasserstoff-reiche Flüssigfraktion abgezogen und dem Abscheider D 3 zugeführt. Am Kopf des Abscheiders D 2 wird über Leitung 13 eine Wasserstoff-reiche Gasfraktion abgezogen und im Wärmetauscher E 2 gegen abzukühlende Verfahrensströme angewärmt. Über Leitung 20 wird die angewärmte Gasfraktion dem Wärmetauscher E 3 zugeführt, in diesem gegen abzukühlende Verfahrensströme weiter erwärmt und letztendlich über Leitung 21 aus der Tieftemperaturzerlegung abgeführt.

Ein Teilstrom der dem Wärmetauscher E 2 über Leitung 13 zugeführten Wasserstoff-reichen Gasfraktion wird über Leitung 14 zunächst einer ersten Entspannungsturbine T 1, über Leitung 15 einer zweiten Entspannungsturbine T 2 und letztendlich über Leitung 16 dem Wärmetauscher E 2 zugeführt, wobei vor bzw. unmittelbar bei der Zuführung in den Wärmetauscher E 2 ein Vermischen mit der Kohlenwasserstoff-reichen Flüssigfraktion in Leitung 5 erfolgt. Die bei der kälte- und arbeitsleistenden Entspannung der Wasserstoff-reichen Gasfraktion in den Entspannungsturbinen T 1 und T 2 freiwerdende Energie kann z. B. zur Stromgewinnung genutzt werden.

Am Kopf des Abscheiders D 3 wird über Leitung 23 ein Methan-reicher Gasstrom abgezogen, der unter Umständen vor die Verdichtungs- und Vorbehandlungsschritte zurückgeführt werden kann. Aus dem Sumpf des Abscheiders D 3 wird eine C₃-Kohlenwasserstoff-reiche Produktfraktion abgezogen, mittels der Pumpe P auf den gewünschte Abgabedruck gepumpt und anschließend über Leitung 25 dem Wärmetauscher E 3 zugeführt. In diesem wird die Produktfraktion gegen abzukühlende Verfahrensströme angewärmt und verdampft und anschließend über Leitung 26 aus der Tieftemperaturzerlegung geführt.

Ein Teilstrom der in der Leitung 25 dem Wärmetauscher E 3 zugeführten Produktfraktion kann über Leitung 27 dem Wärmetauscher E 1 zugeführt und in diesem zur Kältebereitstellung verwendet werden. Aus dem Wärmetauscher E 1 wird dieser Teil der Produktfraktion anschließend über Regelventil e und Leitung 28 wieder der Produktfraktion in Leitung 26 beigemischt.

**Figur 2** zeigt ein zu der Figur 1 weitgehend identisches Verfahren, das sich insbesondere zur Tieftemperaturzerlegung eines im wesentlichen aus Wasserstoff, Methan und C₄-Kohlenwasserstoff-reichen Stromes eignet. Im folgenden sei lediglich auf die Unterschiede zwischen den Verfahrensweisen der Figuren 1 und 2 eingegangen.

Im Falle der Verfahrensweise der Figur 2 wird der Kohlenwasserstoff-reiche Strom, der über Leitung 1 dem Wärmetauscher E 3 zugeführt und in diesem abgekühlt und teilkondensiert wird, anschließend über Leitung 1' einer zusätzlichen Kühleinheit R zugeführt. In dieser wird der Kohlenwasserstoff-reiche Einsatzstrom gegen ein Kältemedium, wie z. B. Propylen oder Propan, weiter abgekühlt und anschließend über Leitung 2 dem Verzweigepunkt, in dem die Aufteilung des Stromes in zwei Teilströme 3 und 4 erfolgt, zugeführt.

Die am Sumpf des zweiten Abscheiders D 2 über Leitung 12 und Ventil d abgezogene C₄-Kohlenwasserstoff-reiche Flüssigfraktion wird nun nicht direkt dem dritten Abscheider D 3 zugeführt. Sie wird stattdessen nach dem Beimischen eines Teilstromes des über Leitung 18 herangeführten, in der Entspannungsturbine T 1 entspannten Wasserstoff-reichen Stromes zunächst im Wärmetauscher E 2 gegen abzukühlende Verfahrensströme angewärmt und teilweise verdampft. Anschließend wird sie der C₃-Kohlenwasserstoff-reichen Flüssigfraktion in der Leitung 30 beigemischt und gelangt zusammen mit dieser oder über eine separate, strichpunktiert gezeichnete Leitung 31 in den dritten Abscheider D 3.

Während bei dem Verfahren gemäß der Figur 1 der über Leitung 14 aus dem Wärmetauscher E 2 abgezogene Teilstrom der Wasserstoff-reichen Gasfraktion zweistufig entspannt wird, erfolgt im Falle des Verfahrens der Figur 2 lediglich eine einstufige Entspannung in der Entspannungsturbine T 1. Daran anschließend wird der entspannte Wasserstoff-reiche Strom in zwei Teilströme aufgeteilt, wobei der erste Teilstrom 17 dem Kohlenwasserstoff-reichen Einsatzstrom in der Leitung 5 beigemischt wird, während der bereits erwähnte zweite Teilstrom des Wasserstoff-reichen Stromes in Leitung 18 mit der C₄-Kohlenwasserstoff-reichen Flüssigfraktion in Leitung 12 vermischt wird.

Die am Kopf des dritten Abscheiders D 3 über Leitung 23 abgezogene Methan-reiche Gasfraktion wird nun ebenfalls im Wärmetausch mit abzukühlenden Verfahrenströmen durch den Wärmetauscher E 3 geführt und anschließend über Leitung 24 aus Tieftemperaturzerlegung abgezogen.

Wie aus den Figuren 1 und 2 ersichtlich, wird der Wärmetauscher E 1 von lediglich drei Verfahrensströmen, nämlich dem im wesentlichen aus Wasserstoff, Methan und C₃-, C₄- oder C₃/C₄-Kohlenwasserstoffen bestehenden Strom in Leitung 8 bzw. 9, dem C₃-, C₄- oder C₃/C₄-Kohlenwasserstoff-reichen Dehydrierungs-Strom in Leitung 6 bzw. 7, dem zumindest ein Teil des Wasserstoffs beigemischt ist, und dem Teilstrom des aus der Tieftemperaturzerlegung abgezogenen, bei der Zerlegung des im wesentlichen aus Wasserstoff, Methan und C₃-, C₄- oder C₃/C₄-Kohlenwasserstoffen bestehenden Stromes gewonnenen, C₃-, C₄- oder C₃/C₄-Kohlenwasserstoff-reichen Stromes in Leitung 27 bzw. 28, durchströmt.

Der Wärmetauscher E 1 kann somit so einfach und symmetrisch wie möglich konzipiert werden, sodaß sowohl die theoretischen Berechnungen bzgl. des Wärmeaustausches sowie die Realisierung des Wärmetauschers E 1 wesentlich vereinfacht werden.

## Patentansprüche

1. Verfahren zur Tieftemperaturzerlegung durch Abkühlung und Teilkondensation eines im Wesentlichen aus Wasserstoff, Methan und C₃-, C₄ oder C₃/C₄-Kohlenwasserstoffen bestehenden Stromes (8), der zuvor eine Dehydrierung durchläuft, wobei die bei der Zerlegung durch Abkühlung und Teilkondensation dieses Stromes gewonnene und von diesem Strom abgetrennte Wasserstoff-reiche Gasfraktion (14) wenigstens teilweise einem zum Zwecke der Kältebereitstellung durch die Tieftemperaturzerlegung geführten, C₃-, C₄- oder C₃/C₄-Kohlenwasserstoff-reichen Dehydrierungs-Einsatzstrom beigemischt wird, während die nicht dem C₃-, C₄- oder C₃/C₄-Kohlenwasserstoff-reichen Dehydrierungs-Einsatzstrom (1) beigemischte Wasserstoff-reiche Gasfraktion (20) und der bei der Zerlegung des im Wesentlichen aus Wasserstoff, Methan und C₃-, C₄- oder C₃/C₄-Kohlenwasserstoffen bestehenden Stromes gewonnene, C₃-, C₄- oder C₃/C₄-Kohlenwasserstoff-reiche Strom (26) aus der Zerlegung nach vorheriger Anwärmung und Verdampfung abgezogen werden, **dadurch gekennzeichnet, dass** die Abkühlung (E1) und Teilkondensation des im Wesentlichen aus Wasserstoff, Methan und C₃-, C₄- oder
C₃/C₄-Kohlenwasserstoffen bestehenden Stromes (8) und die Anwärmung und Verdampfung (E1) des C₃-, C₄- oder C₃/C₄-Kohlenwasserstoff-reichen Dehydrierungs-Einsatz Stromes (6), dem zumindest ein Teil der Wasserstoff-reichen Gasfraktion (3) beigemischt ist, in einem einzigen Wärmetauscher (E1) erfolgt, wobei lediglich ein weiterer Strom (27), der der Kältebereitstellung dient, am Wärmeaustausch (E1) zwischen diesen beiden Verfahrensströmen beteiligt ist, und wobei dieser weitere Strom (27) ein Teilstrom des aus der Tieftemperaturzerlegung abgezogenen, bei der Zerlegung des im Wesentlichen aus Wasserstoff, Methan und C₃-, C₄- oder C₃/C₄-Kohlenwasserstoffen bestehenden Stromes (8) gewonnenen, flüssigen C₃-, C₄- oder C₃/C₄-Kohlenwasserstoff-reichen Stromes (25) ist.

2. Verfahren nach Anspruch 1, **dadurch gekennzeichnet, dass** als Wärmetauscher (E1) für den Wärmeaustausch zwischen dem im Wesentlichen aus Wasserstoff, Methan und C₃-, C₄- oder C₃/C₄-Kohlenwasserstoffen bestehenden Strom (8) und dem C₃-, C₄- oder C₃/C₄-Kohlenwasserstoff-reichen Dehydrierungs-Einsatzstrom (6) ein Plattenwärmetauscher, vorzugsweise ein Aluminium-Plattenwärmetauscher, verwendet wird.

3. Verfahren nach Anspruch 1 oder 2, **dadurch gekennzeichnet, dass** der zum Zwecke der Kältebereitstellung durch die Tieftemperaturzerlegung geführte, C₃-, C₄- oder C₃/C₄-Kohlenwasserstoff-reiche Dehydrierungs-Einsatzstrom (1) auf unterschiedlichen Temperatumiveaus mit der in der Tieftemperaturzerlegung gewonnenen Wasserstoff-reichen Gasfraktion (16, 6) vermischt wird.

## Claims

1. Process for low-temperature fractionation through cooling and partial condensation of a stream (8) essentially consisting of hydrogen, methane and C₃, C₄ or C₃/C₄ hydrocarbons, which stream passes in advance through a dehydrogenation, the hydrogen-rich gas fraction (14) produced in the fractionation through cooling and partial condensation of this stream and separated off from this stream being at least in part admixed, for the purposes of providing refrigeration, with a C₃, C₄ or C₃/C₄ hydrocarbon-rich dehydrogenation feed stream conducted through the low-temperature fractionation, while the hydrogen-rich gas fraction (20) which is not admixed with the C₃, C₄ or C₃/C₄ hydrocarbon-rich dehydrogenation feed stream (1) and the C₃, C₄ or C₃/C₄ hydrocarbon-rich stream (26) produced in the fractionation of the stream essentially consisting of hydrogen, methane and C₃, C₄ or C₃/C₄ hydrocarbons are taken off from the fractionation after prior heating and evaporation, **characterized in that** the cooling (E1) and partial condensation of the stream (8) essentially consisting of hydrogen, methane and C₃, C₄ or C₃/C₄ hydrocarbons and the heating and evaporation (E1) of the C₃, C₄ or C₃/C₄ hydrocarbon-rich dehydrogenation feed stream (6), to which at least some of the hydrogen-rich gas fraction (3) is added, is performed in a single heat exchanger (E1), only one further stream (27) participating in the heat exchange (E1), which serves to provide refrigeration, between these two process streams, and this further stream (27) being a partial stream of the liquid C₃, C₄ or C₃/C₄ hydrocarbon-rich stream (25) produced in the fractionation of the stream (8) essentially consisting of hydrogen, methane and C₃, C₄ or C₃/C₄ hydrocarbons and taken off from the low-temperature fractionation.

2. Process according to Claim 1, **characterized in that** the heat exchanger (E1) used for the heat exchange between the stream (8) essentially consisting of hydrogen, methane and C₃, C₄ or C₃/C₄ hydrocarbons and the C₃, C₄ or C₃/C₄ hydrocarbon-rich dehydrogenation feed stream (6) is a plate heat exchanger, preferably an aluminium plate heat exchanger.

3. Process according to Claim 1 or 2, **characterized in that** the C₃, C₄ or C₃/C₄ hydrocarbon-rich dehydrogenation feed stream (1) conducted through the low-temperature fractionation for the purpose of providing refrigeration is mixed at different temperature levels with the hydrogen-rich gas fraction (16, 6) produced in the low-temperature fractionation.

## Revendications

1. Procédé pour le fractionnement à basse température, par refroidissement et condensation partielle, d'un courant (8) consistant essentiellement en hydrogène, méthane et hydrocarbures en C₃, C₄ ou C₃/C₄, précédemment soumis à une déshydrogénation, la fraction gazeuse (14) riche en hydrogène et séparée de ce courant, obtenue lors du fractionnement par refroidissement et condensation partielle de ce courant, étant ajoutée au moins en partie à un courant d'alimentation de déshydrogénation riche en hydrocarbures en C₃, C₄ ou C₃/C₄, envoyé dans le but du refroidissement dans le fractionnement à basse température, tandis que la fraction gazeuse (20) riche en hydrogène, non ajoutée au courant d'alimentation (1) de déshydrogénation riche en hydrocarbures en C₃, C₄ ou C₃/C₄ et le courant (26) riche en hydrocarbures en C₃, C₄ ou C₃/C₄, obtenu lors du fractionnement du courant consistant essentiellement en hydrogène, méthane et hydrocarbures en C₃, C₄ ou C₃/C₄ sont évacués du fractionnement après chauffage et vaporisation préliminaires, **caractérisé en ce que** le refroidissement (E1) et la condensation partielle du courant (8) consistant essentiellement en hydrogène, méthane et hydrocarbures en C₃, C₄ ou C₃/C₄ et le chauffage et la vaporisation (E1) du courant d'alimentation (6) de déshydrogénation riche en hydrocarbures en C₃, C₄ ou C₃/C₄, auquel est ajoutée au moins une partie de la fraction (3) riche en hydrogène, s'effectuent dans unique échangeur thermique (E1), un seul autre courant (27) qui sert au refroidissement, participant à l'échange thermique (E1) entre ces deux courants de processus, et cet autre courant (27) étant un courant partiel du courant liquide (25) riche en hydrocarbures en C₃, C₄ ou C₃/C₄, obtenu lors du fractionnement du courant (8) consistant essentiellement en hydrogène, méthane et hydrocarbures en C₃, C₄ ou C₃/C₄.

2. Procédé selon la revendication 1, **caractérisé en ce que** comme échangeur thermique (E1) pour l'échange thermique entre le courant (8) consistant essentiellement en hydrogène, méthane et hydrocarbures en C₃, C₄ ou C₃/C₄ et le courant d'alimentation (6) de déshydrogénation riche en hydrocarbures en C₃, C₄ ou C₃/C₄, on utilise un échangeur thermique à plaques, de préférence un échangeur thermique à plaques en aluminium.

3. Procédé selon la revendication 1 ou 2, **caractérisé en ce que** le courant d'alimentation (1) de déshydrogénation riche en hydrocarbures en C₃, C₄ ou C₃/C₄, envoyé dans le but du refroidissement dans le fractionnement à basse température, est mélangé à différents niveaux de température avec la fraction (16, 6) riche en hydrogène, obtenue dans le fractionnement à basse température.
